(19) Europäisches Patentamt
European Patent Office
Office européen des brevets

(11) **EP 3 209 348 B1**

(12) **EUROPÄISCHE PATENTSCHRIFT**

(45) Veröffentlichungstag und Bekanntmachung des Hinweises auf die Patenterteilung:
**30.06.2021 Patentblatt 2021/26**

(21) Anmeldenummer: **15781340.3**

(22) Anmeldetag: **14.10.2015**

(51) Int Cl.:
*A61M 1/16* (2006.01)

(86) Internationale Anmeldenummer:
**PCT/EP2015/073792**

(87) Internationale Veröffentlichungsnummer:
**WO 2016/062593 (28.04.2016 Gazette 2016/17)**

(54) **VORRICHTUNG ZUR VERSORGUNG EINER DIALYSEVORRICHTUNG MIT DIALYSIERFLÜSSIGKEIT**

DEVICE FOR SUPPLYING DIALYSIS LIQUID TO A DIALYSIS APPARATUS

DISPOSITIF POUR ALIMENTER UN APPAREIL DE DIALYSE EN LIQUIDE DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.10.2014 DE 102014015858**

(43) Veröffentlichungstag der Anmeldung:
**30.08.2017 Patentblatt 2017/35**

(73) Patentinhaber: **Fresenius Medical Care Deutschland GmbH
61352 Bad Homburg (DE)**

(72) Erfinder:
• **JONAS, Jörg
Bogotá (CO)**

• **WEHMEYER, Wolfgang
72076 Tübingen (DE)**

(74) Vertreter: **Oppermann, Frank
OANDO Oppermann & Oppermann LLP
Wilhelminenstrasse 1a
65193 Wiesbaden (DE)**

(56) Entgegenhaltungen:
**EP-A1- 2 564 884       DE-A1-102009 037 917
DE-B3- 10 302 691      DE-U1- 9 320 151
US-A1- 2013 037 485   US-A1- 2014 209 520**

• **None**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 209 348 B1

## Beschreibung

Die Erfindung betrifft eine Vorrichtung zur Versorgung einer Dialysevorrichtung mit Dialysierflüssigkeit sowie eine Dialysevorrichtung mit einer Vorrichtung zur Versorgung der Dialysevorrichtung mit Dialysierflüssigkeit.

Während der Dialyse strömt durch die Blutkammer des Dialysators kontinuierlich Blut des Patienten, während durch die Dialysierflüssigkeitskammer fortlaufend Dialysierflüssigkeit fließt. Zur Herstellung von Dialysierflüssigkeit können vorgefertigte Dialysierflüssigkeitskonzentrate verwendet werden, die in den Dialysevorrichtungen mit Wasser verdünnt werden. In Dialysezentren werden Dialysierflüssigkeitskonzentrate entweder als vorgefertigtes Produkt in Kanistern, Beuteln oder Kartuschen zur Verfügung gestellt oder über ein Ringleitungssytem aus einem zentralen Tank bereitgestellt.

Zentral zur Verfügung gestellte Dialysierflüssigkeitskonzentrate sind für den Anwender einfach zu handhaben, sie haben aber den Nachteil, dass die Dialysierflüssigkeit nicht individuell auf die Bedürfnisse des Patienten abgestimmt werden kann. Dezentral bereitgestellte Konzentrate erlauben zwar eine individuelle Anpassung der Dialysierflüssigkeit an den Patienten, sie müssen aber für jede einzelne Dialysebehandlung an die Dialysevorrichtung gebracht werden.

In der Dialyse finden für die Herstellung eines flüssigen Dialysekonzentrats Beutel oder Kartuschen Verwendung, die mit einem pulverförmigen Dialysierflüssigkeitskonzentrat befüllt sind. Die Konzentratbeutel oder -kartuschen enthalten eine Menge an pulverförmigen Dialysierflüssigkeitskonzentrat, die für eine einzige Dialysebehandlung ausreichend ist. Die Beutel oder Kartuschen sind mit Bikarbonat befüllt. Handelsübliche Bikarbonatbeutel enthalten 650 bis 950g Natriumbicarbonat. Aus dem pulverförmigen Bikarbonat-Konzentrat wird zunächst ein flüssiges Bikarbonat-Konzentrat hergestellt. Für die Herstellung der Dialysierflüssigkeit ist ein weiteres Säurekonzentrat erforderlich, das in einem Kanister oder mit einer zentralen Versorgung bereitgestellt wird. Bikarbonat-Konzentrat und Säurekonzentrat werden dann mit Wasser zu der fertigen Dialysierflüssigkeit gemischt.

Die bekannten Konzentratbeutel mit einem pulverförmigen Dialysierflüssigkeitskonzentrat sind nur zur einmaligen Verwendung für nur eine Dialysebehandlung bestimmt. Wenn die vorkonfektionierten Trockenkonzentrate, die nur für eine Behandlung bereitgestellt werden, in der Praxis aber nicht aufgebraucht werden, müssen die nach der Behandlung in den Beuteln befindlichen Restmengen verworfen werden. Eine korrekte Entsorgung der Verpackungsmaterialien durch Granulieren oder Verbrennen ist nur nach einer vollständigen Entleerung der Beutel möglich.

Die DE 103 02 691 B3 beschreibt eine Vorrichtung zur Versorgung einer Dialysevorrichtung mit Dialysierflüssigkeit, bei der Bikarbonat-Konzentrat für eine Dialysebehandlung in einem Behältnis bereitgestellt wird, das an die Dialysevorrichtung angeschlossen wird. In der Dialysevorrichtung wird das pulverförmige Dialysierflüssigkeitskonzentrat mit Wasser gemischt. Eine Steuer- und Recheneinheit berechnet die für die Dialysebehandlung einzustellende Dialysierflüssigkeitsrate derart, dass nach Ablauf einer vorgegebenen Behandlungszeit eine vorgegebene Restmenge an Dialysierflüssigkeitskonzentrat oder keine Restmenge an Konzentrat in dem Behältnis verbleibt.

Der Erfindung liegt die Aufgabe zugrunde, eine Vorrichtung zu schaffen, mit der eine bedarfsgerechte Bereitstellung von Dialysierflüssigkeit bei vereinfachter Handhabung und reduzierten Herstellungs- und Transportkosten möglich ist. Eine Aufgabe der Erfindung ist auch, eine Dialysevorrichtung mit einer Vorrichtung zur Versorgung der Dialysevorrichtung mit Dialysierflüssigkeit zu schaffen.

Die Lösung dieser Aufgaben erfolgt mit den Merkmalen des unabhängigen Patentanspruchs. Die abhängigen Ansprüche betreffen vorteilhafte Ausführungsformen der Erfindung.

Die erfindungsgemäße Vorrichtung beruht darauf, ein vorgefertigtes Dialysierflüssigkeitskonzentrat in einem zur einmaligen Verwendung bestimmten Behältnis bereitzustellen, dass eine Menge an Konzentrat enthält, die für mehrere Behandlungszyklen ausreichend ist, die nacheinander in einem vorgegebenen Zeitraum durchgeführt werden sollen. Das Dialysierflüssigkeitskonzentrat ist vorzugsweise ein pulverförmiges Dialysierflüssigkeitskonzentrat, das als Trockenkonzentrat bereitgestellt wird. Das Konzentrat kann aber auch flüssig sein. Das Behältnis kann unterschiedlich ausgebildet sein. Vorzugsweise ist das Behältnis ein Beutel oder eine Kartusche. Der vorgegebene Zeitraum, in dem die Behandlungszyklen durchgeführt werden sollen, kann unterschiedlich bemessen sein. Vorzugsweise ist der Zeitraum ein Tag, an dem mehrere Dialysebehandlungen durchgeführt werden, so dass das Behältnis den Gesamtbedarf an Konzentrat für einen Dialysetag enthält.

Die einzelnen Behandlungszyklen umfassen jeweils eine der Dialysebehandlung vorausgehende Vorbereitungsphase zur Vorbereitung der Dialysebehandlung und eine Behandlungsphase zur Durchführung der eigentlichen Dialysebehandlung, wobei die für einen Behandlungszyklus vorgesehene Menge an Dialysierflüssigkeitskonzentrat eine vorgegebene Menge an Dialysierflüssigkeitskonzentrat für die Vorbereitungsphase und eine vorgegebene Menge an Dialysierflüssigkeitskonzentrat für die Behandlungsphase umfasst.

Die Vorbereitungsphase kann sich aus einer Testphase, die im Allgemeinen vorgeschrieben ist, und einer optionalen Reinigungsphase zusammensetzen, in denen bestimmte Mengen an Dialysierflüssigkeitskonzentrat verbraucht werden. Während der Behandlungsphase wird Dialysierflüssigkeitskonzentrat zur Herstellung der Dialyselösung verbraucht. Für die Behandlung kann es erforderlich sein, Dialysierflüssigkeitskonzentrat auch zur Herstellung einer Substitutionslösung für eine Prä- oder Postdilution bereitzustellen. Nachfolgend wird eine Dialyselösung und eine Substitutionslösung auch als Dialysierflüssigkeit bezeichnet.

Wenn eine Mehrzahl von Behältnissen mit unterschiedlichen Füllvolumina bereitgestellt werden, kann aus den zur Verfügung stehenden Behältnissen ein Behältnis ausgewählt werden, das diejenige Menge an Dialysierflüssigkeitskonzentrat enthält, die für die Anzahl von Behandlungszyklen ausreichend ist, die in dem vorgegebenen Zeitraum nacheinander durchgeführt werden sollen. Damit ist die Anzahl der beispielsweise an einem Dialysetag durchzuführenden Dialysebehandlungen nicht auf eine bestimmte Anzahl von Behandlungen beschränkt. Die einzelnen Behältnisse unterscheiden sich vorzugsweise nur in dem Füllvolumen, so dass sie gegeneinander austauschbar sind und nicht unterschiedliche Anschlüsse an der Dialysevorrichtung erfordern. Beispielsweise können Beutel oder Kartuschen mit einer Menge an Dialysierflüssigkeitskonzentrat für drei oder vier Behandlungen an einem Tag, vorzugsweise drei Behandlungen, bereitgestellt werden. Die maximale Anzahl der Behandlungen mit einem Behältnis ergibt sich aus dem zeitlichen Grenzwert für das tolerierbare Keimwachstum in dem Behältnis, der von dem Hersteller ermittelt und als Sicherheitsparameter in der Dialysevorrichtung hinterlegt werden kann.

Das Behältnis, beispielsweise für drei Dialysebehandlungen an einem Dialysetag, wird an die Dialysevorrichtung angeschlossen. Während das Behältnis in dem vorgegebenen Zeitraum an die Dialysevorrichtung angeschlossen ist, wird für die einzelnen Behandlungszyklen die Dialysierflüssigkeit unter Verwendung der jeweiligen Teilmenge an Dialysierflüssigkeitskonzentrat hergestellt. Das Behältnis wird von der Dialysevorrichtung erst nach der Durchführung der vorgegebenen Anzahl von Dialysebehandlungen abgenommen.

Die Bereitstellung des Gesamtbedarfs an Dialysierflüssigkeitskonzentrat für einen Dialysetag in nur einem Behältnis reduziert die Anzahl der Handhabungsschritte vom Auspacken der Behältnisse über den internen Transport im Dialysezentrum bis hin zu dem Anschluss der Behältnisse an die Dialysevorrichtungen und dem Verwerfen der Behältnisse nach der Durchführung der Dialysebehandlungen erheblich. Da das Behältnis am Dialysetag an der Dialysevorrichtung angeschlossen bleibt, kann der Ablauf zwischen den einzelnen Behandlungen am Dialysetag beschleunigt werden. Das Verfahren erlaubt somit einen schnelleren Gesamtablauf der Dialysebehandlungen an einem Dialysetag. Der schnellere Gesamtablauf führt letztlich zu Zeit- und Kosteneinsparungen.

Die Herstellungs- und Transportkosten für die Bereitstellung von Dialysierflüssigkeitskonzentrat werden reduziert, da weniger primäres Verpackungsmaterial bereitgestellt und transportiert werden muss. Darüber hinaus sinken die Kosten für die Entsorgung der Behältnisse und der möglicherweise verbleibenden Restmenge an nicht verbrauchtem Dialysierflüssigkeitskonzentrat. Dadurch werden die Ressourcen von Dialysierflüssigkeitskonzentrat und Wasser während der Behandlungen optimal genutzt. Das Verfahren kann automatisch und ohne Eingriff des Bedienpersonals durchgeführt werden.

Die erfindungsgemäße Vorrichtung sieht die Eingabe von Behandlungsparametern für jeden Behandlungszyklus der vorgegebenen Anzahl von Behandlungszyklen mit einer Eingabeeinheit vor. Die Behandlungsparameter können patientenspezifische Behandlungsparameter, beispielsweise die vom Arzt vorgegebene Dialysedosis, und maschinenspezifische Behandlungsparameter, beispielsweise die Blutflussrate oder Dialysierflüssigkeitsrate umfassen. Auf der Grundlage der eingegebenen Behandlungsparameter wird mit einer Rechen- und Auswerteinheit für jeden Behandlungszyklus der vorgegebenen Anzahl von Behandlungszyklen eine vorgesehene Menge an Dialysierflüssigkeitskonzentrat bestimmt. Die Bestimmung der individuellen Konzentratmengen für die einzelnen Patienten kann nach unterschiedlichen Gesichtspunkten erfolgen. Für die Erfindung ist nicht relevant, wie die individuellen Konzentratmengen von der Rechen- und Auswerteinheit bestimmt werden. Allein entscheidend ist, dass von der Rechen- und Auswerteinheit die Konzentratmengen unter Berücksichtigung von eingegebenen Behandlungsparametern automatisch vorgegeben werden.

Die erfindungsgemäße Vorrichtung erlaubt vor der Durchführung der Behandlung einen Abgleich der in dem Behältnis vorhandenen Konzentratmenge und der geplanten Verbrauchsmenge, die sich aus der Verschreibung des Arztes und den maschinenseitig festgelegten Behandlungsparametern ergibt. Hierzu wird die Differenz von der in dem Behältnis enthaltenden Menge an Dialysierflüssigkeitskonzentrat und der für die vorgegebene Anzahl von Behandlungszyklen vorgesehenen Menge an Dialysierflüssigkeitskonzentrat berechnet. Wenn die in dem Behältnis enthaltende Menge an Dialysierflüssigkeitskonzentrat kleiner als die für die vorgegebene Anzahl von Behandlungszyklen vorgesehene Menge an Dialysierflüssigkeitskonzentrat ist, wird ein Alarmsignal erzeugt, das einen optischen und/oder akustischen und/oder taktilen Alarm auslösen kann. In diesem Zusammenhang wird unter einem Alarm jedes Ereignis verstanden, das den Anwender darauf hinweist, dass die Konzentratmenge nicht ausreichend ist. Der Benutzer kann dann ein anderes Behältnis mit einem größeren Füllvolumen auswählen. Die geplante Konzentratmenge und die vorhandene Restmenge für die nachfolgenden Behandlungen können dem Benutzer auch angezeigt werden.

Wenn die in dem Behältnis enthaltende Menge an Dialysierflüssigkeitskonzentrat hingegen nicht kleiner als die für die vorgegebene Anzahl von Behandlungszyklen vorgesehene Menge an Dialysierflüssigkeitskonzentrat ist, wird ein Steuersignal erzeugt. In diesem Zusammenhang wird unter einem Steuersignal jedes Signal verstanden, das den ersten Behandlungszyklus einleiten kann.

Folglich ist sichergestellt, dass die Dialysebehandlungen nur dann erfolgen können, wenn eine ausreichende Konzentratmenge zur Verfügung steht. Bei einem Überschuss von Dialysierflüssigkeitskonzentrat sieht die erfindungsgemäße Vorrichtung eine Aufteilung des Überschusses unter unterschiedlichen Gesichtspunkten vor. Dabei wird aber angestrebt, dass nach der Durchführung sämtlicher Behandlungszyklen in dem Behältnis kein Dialysierflüssigkeitskonzentrat oder nur eine bestimmte Restmenge an Konzentrat verbleibt.

Bei einer Ausführungsform wird ein möglicher Überschuss an Dialysierflüssigkeitskonzentrat in dem letzten Behandlungszyklus verbraucht, so dass das Behältnis vollständig entleert ist oder als Puffer ein kleines Restvolumen in dem Behältnis verbleibt. Damit kann die Dialysedosis für den letzten Patienten erhöht werden. Bei einer alternativen Ausführungsform wird vor der Durchführung der Behandlungszyklen der Konzentratüberschuss auf sämtliche geplanten Behandlungszyklen verteilt. Die Verteilung kann zu gleichen Teilen oder nach einer vorgegebenen individuellen Gewichtung erfolgen, beispielsweise in Abhängigkeit von der für die Patienten vorgegebenen individuellen Konzentratmengen. Eine größere Konzentratmenge, die mit einer höheren Dialysedosis verbunden ist, kommt damit allen Behandlungen zugute.

Nach der Durchführung der einzelnen Dialysebehandlungen wird laufend überprüft, ob die Konzentratmenge ausreichend ist. Dabei kann überprüft werden, ob die Konzentratmenge für die nachfolgende Behandlung oder für sämtliche noch durchzuführende Behandlungen ausreichend ist. Es kann auch eine Überprüfung erfolgen, ob die Konzentratmenge für die nachfolgende Behandlung und für sämtliche Behandlungen ausreichend ist. Wenn dies nicht der Fall ist, wird wieder ein Alarmsignal zur Erzeugung eines Alarms erzeugt. Ansonsten wird wieder ein Steuersignal zur Einleitung des jeweils nächsten Behandlungszyklus erzeugt.

Zwischen den einzelnen Behandlungszyklen wird laufend überprüft, ob die tatsächlich verbrauchte Menge an Dialysierflüssigkeitskonzentrat der geplanten Konzentratmenge entspricht, um die tatsächliche Menge an Konzentrat für die noch durchzuführenden Behandlungen bestimmen zu können, die sich aus der Differenz der zuvor in dem Behältnis befindlichen und der tatsächlich verbrauchten Menge an Konzentrat ergibt.

Die vor der Durchführung der Behandlungszyklen in dem Behältnis enthaltene Menge an Dialysierflüssigkeitskonzentrat kann von der Rechen- und Auswerteinheit automatisch erkannt werden oder von dem medizinischen Personal mit der Eingabeeinheit eingegeben werden. Die Erfindung sieht verschiedene Varianten vor. Das ausgewählte Behältnis, beispielsweise der Beutel oder die Kartusche, das sich von den anderen Behältnissen in dem Füllvolumen unterscheidet, kann durch ein bestimmtes Identifikationsmerkmal automatisch erkannt werden. Für eine elektrische Identifikation können die bekannten (RFID)-Transponder verwendet werden. Die Daten können aber auch mit einem kapazitiven Messwertaufnehmer aufgenommen werden. Eine optische Identifikation ist mit den bekannten Barcodes möglich. Eine mechanische Identifikation kann mit der Erfassung der Umrisslinien der Behältnisse oder von weiteren Identifikationsmerkmalen, wie Kanten, Rastelementen oder Pins vorgenommen werden. Darüber hinaus ist eine Bestimmung der in dem Behältnis enthaltenen Menge an Dialysierflüssigkeitskonzentrat mit einer gravimetrischen Gewichtsmessung möglich. Des Weiteren ist eine pneumatische Erfassung der Füllmenge durch Bestimmung des Füllvolumens des Behältnisses mit Wasser bis zu einem festgelegten Druckpunkt möglich.

Die erfindungsgemäße Dialysevorrichtung, die über eine Vorrichtung zur Versorgung der Dialysevorrichtung mit Dialysierflüssigkeit verfügt, kann über ein Dialysierflüssigkeitssystem verfügen, das die Durchführung einer Desinfektions- oder Reinigungsphase zwischen den einzelnen Dialysebehandlungen erlaubt. Bei einer bevorzugten Ausführungsform erfolgt die Herstellung der Dialysierflüssigkeit auch während der Desinfektion oder Reinigung. Bei dieser Ausführungsform umfasst das Dialysierflüssigkeitssystem einen ersten Abschnitt, der die Dialysierflüssigkeitskammer eines durch eine semipermeable Membran in eine Blutkammer und die Dialysierflüssigkeitskammer unterteilten Dialysators einschließt, und einen zweiten Abschnitt, der eine Einheit zur Herstellung von Dialysierflüssigkeit aus dem Dialysierflüssigkeitskonzentrat einschließt. Darüber hinaus weist die Dialysevorrichtung eine Einheit zur Desinfektion des Dialysierflüssigkeitssystem auf, wobei der erste und zweite Abschnitt des Dialysierflüssigkeitssystems als voneinander abtrennbare Abschnitte ausgebildet sind, so dass bei einer Abtrennung in dem einen Abschnitt befindliche Flüssigkeit nicht in den anderen Abschnitt gelangen kann. Die Abtrennung des Dialysierflüssigkeitssystems erlaubt die Durchführung der für die Herstellung der Dialysierflüssigkeit erforderlichen Routine zeitgleich mit der Desinfektion der Dialysevorrichtung, so dass der gesamte Verfahrensablauf für die Herstellung der Dialysierflüssigkeit bzw. der Desinfektion zwischen den Behandlungszyklen nicht vollständig unterbrochen werden muss, wodurch wiederum Zeit eingespart wird. Da der hydraulische Teil für die Aufbereitung der Dialysierflüssigkeit von der Desinfektion getrennt ist, kann auch die Desinfektion verkürzt werden.

Zur Abtrennung des Abschnitts von dem Dialysierflüssigkeitssystem, in dem die Herstellung der Dialysierflüssigkeit aus dem Dialysierflüssigkeitskonzentrat erfolgt, können verschiedene Mittel, beispielsweise ein oder mehrere Absperrorgane, Rückschlagventile oder dgl. vorgesehen sein.

Die Dialysevorrichtung kann eine Steuereinheit für die Einheit zur Desinfektion des Dialysierflüssigkeitssystems aufweisen, die derart konfiguriert ist, dass während der Desinfektion der Dialysevorrichtung die Mittel zum Abtrennen des Abschnitts von dem Dialysierflüssigkeitssystem betätigt sind, so dass der Abschnitt des Dialysierflüssigkeitssystems, in dem die Herstellung der Dialysierflüssigkeit aus dem Dialysierflüssigkeitskonzentrat erfolgt, während der Durchführung der Desinfektion der Dialysevorrichtung abgetrennt ist. Die Steuereinheit ist derart konfiguriert, dass während der Desinfektion der Dialysevorrichtung in dem abgetrennten Abschnitt des Dialysierflüssigkeitssystems, in dem die Herstellung der Dialysierflüssigkeit aus dem Dialysierflüssigkeitskonzentrat erfolgt, die Einheit zur Herstellung von Dialysierflüssigkeit eine für die Herstellung der Dialysierflüssigkeit erforderliche Routine durchführt.

Zur Desinfektion kann ein Heißdesinfektions-Verfahren bei einer hohen Temperatur mit reinem Osmosewasser durchgeführt werden, um Chemikalienreste in Toträumen zwischen desinfizierten und nicht desinfizierten Teilen des Dialysierflüssigkeitssystems vollständig und sicher entfernen zu können. Zur lokalen Entkalkung kann eine Spülung mit einem Säurekonzentrat zwischen den einzelnen Dialysebehandlungen durchgeführt werden. Eine Verkalkung der Maschine kann durch geeignete Maßnahmen verhindert werden, beispielsweise durch die Verwendung von Zitrat als starker Säure im Säurekonzentrat oder durch die Beschränkung der Konzentration von Bikarbonat in Abhängigkeit von der Beimischung von Acetat in dem fertigen Dialysat.

Im Folgenden werden Ausführungsbeispiele der erfindungsgemäßen Vorrichtung unter Bezugnahme auf die Zeichnungen näher erläutert.

Es zeigen:

Fig. 1 ein Ausführungsbeispiel der erfindungsgemäßen Dialysevorrichtung in vereinfachter schematischer Darstellung, und

Fig. 2A eine Einheit zum Anschließen eines mit einem Dialysierflüssigkeitskonzentrat befüllten Behältnisses zur Durchführung von mehreren Behandlunsgzyklen an die Dialysevorrichtung von Fig. 1,

Fig. 2B die Einheit zum Anschließen des Behältnisses von Fig. 2A, an die ein Behältnis zur Durchführung von mehreren Behandlungszyklen angeschlossen ist, und

Fig. 2C ein Vorrat an Behältnissen in schematischer Darstellung, die jeweils für mehrere Behandlungszyklen bestimmt sind.

Die Hämodialysevorrichtung weist einen Dialysator 1 auf, der durch eine semipermeable Membran 2 in eine Blutkammer 3 und eine Dialysierflüssigkeitskammer 4 unterteilt ist. Der Einlass der Blutkammer 3 ist mit einem Ende einer Blutzuführleitung 5 verbunden, in die eine Blutpumpe 6 geschaltet ist, während der Auslass der Blutkammer mit einem Ende einer Blutabführleitung 7 verbunden ist. Zu dem Einlass der Dialysierflüssigkeitskammer 4 führt eine Dialysierflüssigkeitszuführleitung 8, und von dem Auslass der Dialysierflüssigkeitskammer geht eine Dialysierflüssigkeitsabführleitung 9 ab, die zu einem Abfluss 10 führt. In die Dialysierflüssigkeitsabführleitung 9 ist eine Dialysierflüssigkeitspumpe 11 geschaltet. Während der Dialysebehandlung strömt Blut des Patienten durch die Blutkammer 3 des Dialysators 1, während im Gegenstrom Dialysierflüssigkeit durch die Dialysierflüssigkeitskammer 4 fließt. Die semipermeable Membran 2 des Dialysators 1 trennt den extrakorporalen Blutkreislauf I der Dialysevorrichtung von dem Dialysierflüssigkeitssystem II.

Die Vorrichtung zur Versorgung des Dialysators 1 mit Dialysierflüssigkeit ist vorzugsweise Bestandteil der Dialysevorrichtung. Sie kann aber auch eine separate Einheit bilden. Im Folgenden wird die Versorgungsvorrichtung im Einzelnen beschrieben.

Zur Herstellung der Dialysierflüssigkeit werden zwei Konzentrate K1, K2 mit Wasser W in einem vorgegebenen Volumenverhältnis gemischt.

Die Versorgungsvorrichtung verfügt über zwei Einheiten zum Anschluss von jeweils einem Behältnis, wobei das eine Behältnis ein Kanister 12 ist, der der bei dem vorliegenden Ausführungsbeispiel mit Säurekonzentrat befüllt ist. Das andere Behältnis ist ein Beutel 13 ist, der bei dem vorliegenden Ausführungsbeispiel mit Natriumbikarbonat als Trockenkonzentrat befüllt ist.

Von dem Kanister 12 geht eine erste Konzentratleitung 14 ab, die zu einer Mischkammer 15 führt, und von dem Beutel 13 geht eine zweite Konzentratleitung 16 ab, die zu der Mischkammer 15 führt. In die erste und zweite Konzentratleitung 14, 16 sind jeweils eine Proportionierungspumpe 17, 18 geschaltet. Zu der Mischkammer 15 führt ferner eine Wasserleitung 19, die an einer Wasserquelle 38 angeschlossen ist. In die Wasserleitung 19 ist ebenfalls eine Proportionierungspumpe 20 geschaltet.

Die Proportionierungspumpen 17, 18 und 20 sind über Daten- und Steuerleitungen 21, 22 und 23 an eine Rechen- und Auswerteinheit 24 der Versorgungsvorrichtung angeschlossen, die für die Proportionierungspumpen bestimmte Förderraten vorgibt, so dass die Konzentrate K1 bzw. K2 und Wasser jeweils in einem vorgegebenen Volumenverhältnis zur Herstellung der Dialysierflüssigkeit in der Mischkammer 15 gemischt werden. An dem Beutel 13 mit dem Trockenkonzentrat ist ein Wasseranschluss 46 zum Zuführen einer vorgegebenen Menge an Wasser vorgesehen.

Um aus dem Trockenkonzentrat ein mit Wasser in einem vorgegebenen Volumenverhältnis zu mischendes Flüssigkonzentrat zu gewinnen, wird das Pulver zunächst in Wasser gelöst, das aus dem Wasseranschluss 46 in den Beutel 13 zufließt. Durch Zuführung von Wasser wird aus dem pulverförmigen Dialysierflüssigkeitskonzentrat eine gesättigte Lösung hergestellt. Die Herstellung der gesättigten Lösung kann in einem kontinuierlichen Prozess erfolgen.

Das Volumen des aus dem pulverförmigen Dialysierflüssigkeitskonzentrat hergestellten flüssigen Dialysierflüssigkeitskonzentrats ergibt sich aus der Menge des pulverförmigen Dialysierflüssigkeitskonzentrats und des Volumen des zugeführten Wassers, wobei durch die Maschinenparameter ein vorgegebenes Verhältnis zwischen der Menge des pulverförmigen Dialysierflüssigkeitskonzentrats und des Volumens des zugeführten Wassers festgelegt ist.

Von dem Kanister 12 geht eine Entleerungsleitung 26 ab, die zu einer zweiten Mischkammer 27 führt, während von dem Beutel 13 eine zweite Entleerungsleitung 28 abgeht, die zu der Mischkammer 27 führt. Von der Wasserquelle 38 geht eine Wasserleitung 29 ab, die ebenfalls zu der Mischkammer 27 führt. In die erste und zweite Entleerungsleitung 26, 28 und in die Wasserleitung 29 sind jeweils Pumpen 30, 31, 32 geschaltet, die über Steuerleitungen 33, 34, 35 mit der Rechen- und Auswerteinheit 24 verbunden sind. Von der Mischkammer 27 geht eine Ablaufleitung 36 ab, die zu dem Abfluss 10 führt.

Darüber hinaus verfügt die Versorgungsvorrichtung über eine Eingabeeinheit 25, die über eine Datenleitung 39 mit der Rechen- und Auswerteinheit 24 kommuniziert. Auf der Eingabeeinheit 25 können verschiedene Behandlungsparameter eingegeben werden, die sich aus der Verschreibung des Arztes und maschinenseitig festgelegten Behandlungsparametern ergeben. Der Arzt kann z.B. eine Dialysedosis vorgeben, indem er mit der Eingabeeinheit 25 für einen bestimmten Dialysator 1 den Blutfluss, den Dialysierflüssigkeitsfluss und die Behandlungszeit eingibt. Der Arzt kann auch eine Prä- oder Postdilution mit einer vorgegebenen Flussrate für die Substitutionsflüssigkeit verschreiben. Für den Dialysatfluss kann auch ein Profil eingegeben werden. Es sind aber auch andere dem Fachmann bekannte Methoden möglich, um eine Abschätzung des zur Erzielung einer bestimmten Dialysedosis notwendigen Volumens vorzunehmen. Die Menge an Substitutionsflüssigkeit kann beispielsweise aus dem zu behandelnden Blutvolumen bestimmt werden, wobei das gesamte Infusionsvolumen beispielsweise bei Postdilution 30% und bei Prädilution 60% des Blutvolumens betragen kann. Das Blutvolumen wiederum kann aus der vorgegebenen Blutflussrate und dem sich daraus ergebenden effektiven Blutfluss und der vorgegebenen Behandlungszeit berechnet werden.

Für die Unterbrechung der Dialysebehandlung, beispielsweise beim Auftreten von Komplikationen oder für die Durchführung eines Tests oder anderer Routinen zur Vorbereitung der Dialysebehandlung, können eine Bypassleitung 41, in die ein Bypassventil 40 geschaltet ist, und ein Absperrventil 42 stromauf und ein Absperrventil 43 stromab der Dialysierflüssigkeitskammer 4 des Dialysators 1 vorgesehen. Wenn die Dialysebehandlung unterbrochen ist, strömt die Dialysierflüssigkeit durch die Bypassleitung 40 in den Ablauf 10, wobei die Dialysierflüssigkeitskammer 4 von Dialysierflüssigkeit nicht durchflossen wird.

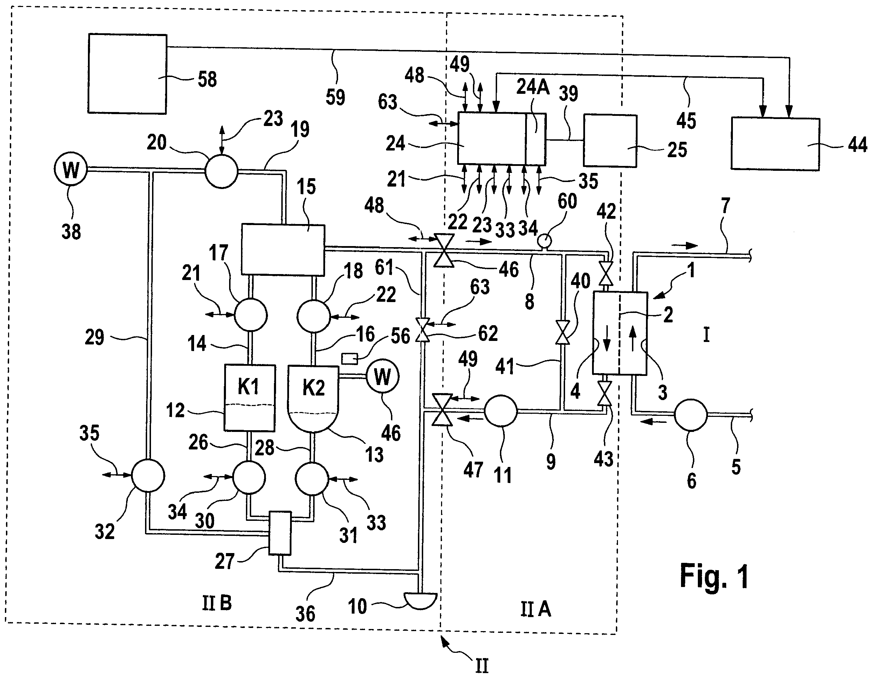

Die Dialysevorrichtung verfügt über eine zentrale Rechen- und Steuereinheit 44, die über eine Datenleitung 45 mit der Rechen- und Auswerteinheit 24 der Versorgungsvorrichtung kommuniziert. Die Rechen- und Auswerteinheit 24 der Versorgungsvorrichtung kann aber auch Bestandteil der zentrale Rechen- und Steuereinheit 44 der Dialysevorrichtung sein. Es können noch weitere Einheiten vorgesehen sein, beispielsweise eine Bilanziereinheit, die aber in Fig. 1 nicht dargestellt sind.

Zur Herstellung der Dialysierflüssigkeit, die dem Dialysator 1 zugeführt werden soll, gibt die Steuer- und Recheneinheit 24 die Förderraten der Proportionierungspumpen 17, 18, 20 derart vor, dass in der Mischkammer 15 die Konzentrate K1, K2 jeweils mit Wasser in dem vorgegebenen Volumenverhältnis gemischt werden.

Die Dialysierflüssigkeit soll beispielsweise aus dem in dem Behältnis 13 zur Verfügung stehenden Dialysierflüssigkeitskonzentrat für drei Behandlungszyklen hergestellt, die an einem Dialysetag aufeinander folgen sollen.

Ein Behandlungszyklus setzt sich zusammen aus einer obligatorischen Testphase und einer optionalen Reinigungsphase sowie der eigentlichen Behandlungsphase. Während der Reinigungsphase kann die Herstellung der Dialysierflüssigkeit mit einem geringen Dialysatfluss weiterlaufen. Die Herstellung der Dialysierflüssigkeit kann aber auch während der Reinigung unterbrochen werden. Während der Behandlung kann die Herstellung von Dialysierflüssigkeit als Substitutionslösung erforderlich sein, wenn der Arzt eine Prä- oder Postdilution verschrieben hat.

Folglich setzt sich die für einen Behandlungszyklus erforderliche Menge an Dialysierflüssigkeit zusammen aus der Menge an Dialysierflüssigkeitskonzentrat für die obligatorische Testphase $V_{Test}$, der Menge an Dialysierflüssigkeitskonzentrat für die optionale Reinigungsphase $V_{Rein}$ und der Konzentratmenge für die Behandlungsphase $V_{Beh}$.

Die für die Behandlungsphase bereitzustellende Konzentratmenge $V_{Beh}$ kann in eine Konzentratmenge zur Bereitstellung der Dialyselösung $V_{Dial}$ (obligatorisch) und in eine Konzentratmenge zur Bereitstellung der Substitutionslösung für die Prä- bzw. Postdilution $V_{Sub}$ (optional) aufgeteilt werden. Bei dem vorliegenden Ausführungsbeispiel sind $V_{Test}$ und $V_{Rein}$ in der Dialysemaschine hinterlegte Behandlungsparameter. $V_{Beh}$ wird von der Rechen- und Auswerteinheit 24 auf der Grundlage der mit der Eingabeeinheit 25 eingegebenen Behandlungsparameter entsprechend der Verschreibung des Arztes bestimmt.

Während einer initialen Testphase kann die Steuer- und Recheneinheit 44 der Dialysevorrichtung die Absperrventile 42, 43 schließen und das Bypassventil 41 öffnen, so dass die Dialysierflüssigkeit durch die Bypassleitung 40 für ein vorgegebenes Zeitintervall $T_{test}$ in den Ablauf 10 strömt. Die Dialysierflüssigkeitsrate beträgt beispielsweise $Qd_{test}$. Nach Ablauf des vorgegebenen Zeitintervalls $T_{test}$ kann dann die eigentliche Dialysebehandlung beginnen. Während der Dialysebehandlung kann eine vorgegebene Dialysierflüssigkeitsrate Qd eingestellt werden. Für den Fall, dass während der Dialysebehandlung eine Komplikation auftritt, kann der Dialysator 1 abgetrennt und die Dialysierflüssigkeit über den Bypass 40 in den Ablauf 10 verworfen werden. Wenn häufig Störungen auftreten, kann es erforderlich sein, die Behandlung entsprechend zu verlängern, um die effektive Behandlungszeit $T_{eff}$ zu erreichen.

Darüber hinaus verfügt die Dialysevorrichtung über eine Einheit 58 zur Desinfektion des Dialysierflüssigkeitssystems II, die in Fig. 1 nur andeutungsweise dargestellt ist. Die Desinfektionseinheit 58 ist über eine Steuerleitung 59 mit der zentralen Steuereinheit 44 der Dialysevorrichtung verbunden. Derartige Desinfektionsvorrichtungen gehören zum Stand der Technik. Zur Desinfektion wird das Desinfektionsmittel, beispielsweise heißes Wasser, über einen Anschluss 60 der Dialysierflüssigkeitszuführleitung 8 zugeführt, wobei die Anschlüsse des Dialysators 1 mit einem nicht dargestellten Kurzschlussstück kurzgeschlossen werden.

Die standardmäßige Desinfektion der Dialysevorrichtungen schließt die Mischkammer 15 ein. Das erfindungsgemäße Verfahren sieht aber eine Modifizierung des Reinigungsverfahrens und der Dialysevorrichtung vor, da das Behältnis 13 zwischen den Behandlungszyklen an der Dialysevorrichtung angeschlossen bleibt.

Das Dialysierflüssigkeitssystem II der Dialysevorrichtung umfasst einen ersten Abschnitt IIA, der die Dialysierflüssigkeitskammer 4 des Dialysators 1 einschließt, und einen zweiten Abschnitt IIB, der die aus den zuvor beschriebenen Komponenten bestehende Einheit zur Herstellung der Dialysierflüssigkeit einschließt. Die beiden Abschnitte IIA und IIB können miteinander verbunden und voneinander getrennt werden. Zum Abtrennen des zweiten Abschnitts IIB, in dem die Herstellung der Dialysierflüssigkeit erfolgt, sind geeignete Mittel vorgesehen, bei denen es sich beispielsweise um in der Dialysierflüssigkeitszuführleitung 8 und Dialysierflüssigkeitsabführleitung 9 angeordnete Absperrorgane 46, 47 handeln kann, die über Steuerleitungen 48, 49 mit der Steuer- und Recheneinheit 24 verbunden sind. Die Darstellung dieser Absperrorgane dient aber hier nur der Illustration. Darüber hinaus ist zwischen der Dialysierflüssigkeitszuführ- 8 und - abführleitung 9 eine Bypassleitung 61 mit einem Bypassventil 62 vorgesehen, das über eine Steuerleitung mit der Rechen- und Auswerteinheit 24 verbunden ist.

Während der Reinigungsphase, in der die Absperrventile 46 und 47 geschlossen sind, ist das Bypassventil 62 geöffnet, wobei die Herstellung der Dialysierflüssigkeit nicht unterbrochen ist. Die Dialysierflüssigkeit, die mit einer möglichst geringen Füssigkeitsrate hergestellt werden kann, wird über die Bypassleitung 61 verworfen.

Die erfindungsgemäße Vorrichtung ist für die Durchführung von mehreren, d.h. mindestens zwei Behandlungszyklen innerhalb eines vorgegebenen Zeitraums, beispielsweise eines Dialysetages, unter Verwendung nur eines Behältnisses 13 bestimmt, das mit einer Menge $M_0$ eines pulverförmigen Dialysierflüssigkeitskonzentrats befüllt ist, die für die vorgegebene Anzahl x von Behandlungszyklen ausreichend ist. Dabei wird vorausgesetzt, dass auch in dem anderen Behältnis 12, in dem das Säurekonzentrat bereitgestellt wird, eine für die vorgegebene Anzahl von Dialysebehandlungen ausreichende Menge an Konzentrat enthalten ist.

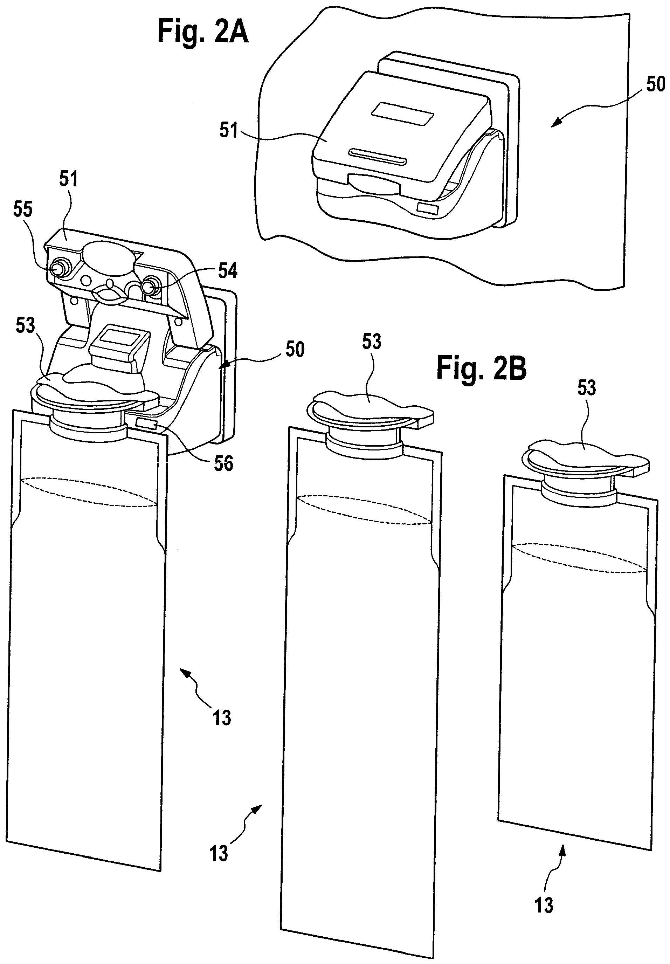

Fig. 2A zeigt die Einheit 50 zum Anschluss des mit dem pulverförmigen Dialysierflüssigkeitskonzentrat befüllten Behältnisses 13. Die Anschlusseinheit 50 weist eine Klappe 51 auf, die in der in Fig. 2A dargestellten Position geschlossen ist. Zum Anschluss des Konzentratbehältnisses 13 wird die Klappe 51 geöffnet. Fig. 2B zeigt die Anschlusseinheit 50 mit geöffneter Klappe 51.

Bei dem vorliegenden Ausführungsbeispiel ist das Konzentratbehältnis 13 ein mit einem Trockenkonzentrat, insbesondere Bikarbonat, befüllter Beutel. Das Konzentratbehältnis kann aber auch eine Kartusche sein. Der Konzentratbeutel 13 weist ein Anschlussstück 52 auf, mit dem sich der Beutel an der Anschlusseinheit 50 bei geöffneter Klappe 51 einhängen lässt. Die Klappe 51 weist an der Unterseite einen Zulaufanschluss 54 für Wasser und einen Ablaufanschluss 55 für das flüssige Dialysierflüssigkeitskonzentrat auf, mit denen bei geschlossener Klappe 51 eine Verbindung zum Beutelinneren hergestellt werden kann.

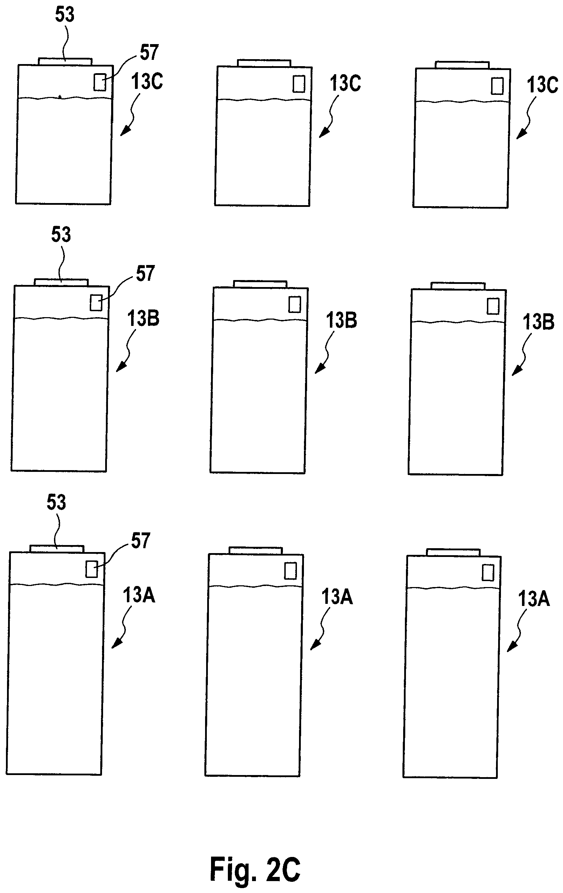

Für die Durchführung der Behandlungszyklen an einem Dialysetag wird eine Mehrzahl von derartigen Konzentratbeuteln 13 zur Verfügung gestellt, die sich nur in der Beutelgröße und dem Füllvolumen unterscheiden. Fig. 2C zeigt mehrere identische Konzentratbeutel 13A, die mit einer ausreichenden Menge an Bikarbonaten für beispielsweise 4 Dialysebehandlungen befüllt sind, sowie mehrere identische Beutel 13B für 3 Behandlungen und mehrere Beutel 13C für nur 2 Behandlungen. Bei der Bemessung der Konzentratmenge in den Beuteln wird von zuvor ermittelten Durchschnittswerten für den Konzentratverbrauch pro Behandlungszyklus ausgegangen.

Für die Durchführung von mehreren Behandlungszyklen an einem Dialysetag wird ein Konzentratbeutel 13 ausgewählt, der die notwendige Menge $M_0$ an Dialysierflüssigkeitskonzentrat enthält. Für einen Dialysetag mit x Behandlungszyklen (x=1-4), beispielsweise mit 3 Behandlungszyklen, wird der Konzentratbeutel 13B ausgewählt, der Bikarbonat für die Anzahl der Behandlungszyklen, beispielsweise 3 Zyklen, enthält. Dieser Konzentratbeutel 13B wird dann an die Anschlusseinheit 50 der Dialysevorrichtung angeschlossen.

Die für die Behandlung vorgegebenen Behandlungsparameter, beispielsweise die Dialysedosis, Blutflussrate, Dialysierflüssigkeitsrate, Substitutionsrate, werden mit der Eingabeeinheit 24 eingegeben.

Die Versorgungsvorrichtung verfügt über eine nur schematisch dargestellte Einheit 56 zur Identifikation des Konzentratbeutels 13B als ein für eine vorgegebene Anzahl von Behandlungszyklen bestimmter Beutel, d.h. ein Beutel für 3 Behandlungszyklen. Die Einheit 56 zur Identifikation des Beutels ist bei dem vorliegenden Ausführungsbeispiel eine optische Leseeinheit für einen auf dem Beutel befindlichen Barcode 57. Es ist aber auch möglich, die vorgegebene Anzahl von Behandlungszyklen mit der Eingabeeinheit 25 einzugeben.

Die Rechen- und Auswerteinheit 24 ist derart konfiguriert, dass sie die nachfolgenden Verfahrensschritte ausführt. Hierzu kann die Rechen- und Auswerteinheit 24 eine Datenverarbeitungseinheit aufweisen, auf der ein Datenverarbeitungsprogramm (Software) läuft.

Die Rechen- und Auswerteinheit 24 berechnet auf der Grundlage der mit der Eingabeeinheit 25 eingegebenen Behandlungsparameter den zu erwartenden Konzentratverbrauch pro Zyklus $V_1, V_2...V_x$:

$$V_1 = V_{Test} + V_{Beh} + V_{Rein(optional)} \text{ (Behandlungsparamter für Zyklus 1)}$$

$$V_2 = V_{Test} + V_{Beh} + V_{Rein(optional)} \text{ (Behandlungsparamter für Zyklus 2)}$$

$$...$$

$$V_x = V_{Test} + V_{Beh} + V_{Rein(optional)} \text{ (Behandlungsparamter für Zyklus x)}$$

Bei dem vorliegenden Ausführungsbeispiel werden für die 3 Behandlungszyklen folgende Konzentratmengen bestimmt:

$$V_1 = V_{Test} + V_{Beh} + V_{Rein(optional)} \text{ (Behandlungsparamter für Zyklus 1)}$$

$$V_2 = V_{Test} + V_{Beh} + V_{Rein(optional)} \text{ (Behandlungsparamter für Zyklus 2)}$$

$$V_3 = V_{Test} + V_{Beh} + V_{Rein(optional)} \text{ (Behandlungsparamter für Zyklus 3)}$$

Daraufhin berechnet die Rechen- und Auswerteinheit 24 die Differenz von der in dem Behältnis 13 enthaltenden Menge $M_0$ an Dialysierflüssigkeitskonzentrat und der für die vorgegebene Anzahl x=3 von Behandlungszyklen vorgesehene Menge $V_1 + V_2 + V_3 ... + V_x$ an Dialysierflüssigkeitskonzentrat:

$$V = V_1 + V_2 + V_3 ... + V_x$$

Bei dem Ausführungsbeispiel wird $V = V_1 + V_2 + V_3$ berechnet. Wenn die in dem Behältnis enthaltende Menge $M_0$ an Dialysierflüssigkeitskonzentrat kleiner als die für die vorgegebene Anzahl von Behandlungszyklen vorgesehene Menge V an Dialysierflüssigkeitskonzentrat ist, wird ein Alarmsignal zur Erzeugung eines Alarms erzeugt. Ein Steuersignal zur Einleitung des ersten Behandlungszyklus wird hingegen erzeugt, wenn die in dem Behältnis enthaltende Menge $M_0$ an Dialysierflüssigkeitskonzentrat nicht kleiner als die für die vorgegebene Anzahl von Behandlungszyklen vorgesehene Menge V ist. Die Steuereinheit 44 der Dialysevorrichtung empfängt das Alarmsignal und das Steuersignal der Versorgungseinrichtung. Wenn ein Alarmsignal erzeugt wird, wird ein optischer und/oder akustischer und/oder taktiler Alarm gegeben. Beispielsweise wird auf einer Anzeigeeinheit 24A der Rechen- und Auswerteinheit 24 dem medizinischen Personal angezeigt, dass die Konzentratmenge nicht ausreichend ist. Auf der Anzeigeeinheit 24A kann auch die in dem Behältnis enthaltende Konzentratmenge $M_0$ und die erforderliche Konzentratmenge V angezeigt werden.

Wenn die Steuereinheit 44 das Steuersignal empfangen hat, beginnt der erste Behandlungszyklus, für den die Konzentratmenge $V_1$ zur Verfügung steht.

In der Praxis kann es vorkommen, dass die für den Behandlungszyklus vorgesehene Konzentratmenge von der tatsächlich verbrauchten Konzentratmenge abweicht. Nach der Durchführung des ersten Behandlungszyklus x=1 und vor der Durchführung des zweiten Behandlungszyklus x=2 überprüft die Rechen- und Auswerteinheit 24, ob das für die noch durchzuführenden Behandlungszyklen zur Verfügung stehende Restvolumen an Dialysierflüssigkeitskonzentrat für den zweiten und dritten Behandlungszyklus ausreichend ist. Zunächst wird die tatsächlich verbrauchte Menge $V_{1,ist}$ an Dialysierflüssigkeitskonzentrat ermittelt und aus der Differenz der vor der Durchführung des ersten Behandlungszyklus in dem Behältnis enthaltenden Menge $M_0$ an Dialysierflüssigkeitskonzentrat und der in dem ersten Zyklus tatsächlich verbrauchten Konzentratmenge $V_{1,ist}$ wird die für den zweiten und dritten Behandlungszyklus noch zur Verfügung stehende Restmenge $M_1$ an Dialysierflüssigkeitskonzentrat in dem Behältnis berechnet:

$$M_1 = M_0 - V_{1,ist}$$

Wenn die Restmenge $M_1$ an Dialysierflüssigkeitskonzentrat kleiner als die für den zweiten und dritten Behandlungszyklus vorgesehene Menge $V_2 + V_3$ an Dialysierflüssigkeitskonzentrat ist, wird ein Alarmsignal erzeugt. Ist die Restmenge $M_1$ hingegen nicht kleiner als die für den zweiten und dritten Behandlungszyklus vorgesehene Menge, wird ein Steuersignal zur Einleitung des zweiten Behandlungszyklus erzeugt.

Nach der Durchführung des zweiten Behandlungszyklus x=2 und vor der Durchführung des dritten Behandlungszyklus x=3 ermittelt die Rechen- und Auswerteinheit 24 die in dem zweiten Behandlungszyklus tatsächlich verbrauchte Menge $V_{2,ist}$ an Dialysierflüssigkeitskonzentrat und berechnet die für den dritten Behandlungszyklus tatsächlich noch zur Verfügung stehende Restmenge an Dialysierflüssigkeitskonzentrat $M_2$:

$$M_2 = M_1 - V_{2,ist}$$

Die Rechen- und Auswerteinheit 24 überprüft, ob die Konzentratrestmenge $M_2$ für den dritten Behandlungszyklus x=3 ausreichend ist. Wenn $M_2$ kleiner als $V_3$ ist, wird ein Alarmsignal erzeugt und wenn $M_2$ nicht kleiner als $V_3$ ist, wird ein Steuersignal zur Einleitung des dritten Behandlungszyklus erzeugt.

In der Praxis wird sich vor der Durchführung des letzten Behandlungszyklus ein Überschuss an Dialysierflüssigkeitskonzentrat ergeben, der für die letzte Behandlung nicht benötigt wird.

Für das Ausführungsbeispiel berechnet sich der Konzentratüberschuss $M_R$:

$$M_R = M_2 - V_3$$

Wenn die Restmenge $M_R$ an Dialysierflüssigkeitskonzentrat größer als die für den letzten Behandlungszyklus $V_3$ vorgesehene Menge an Dialysierflüssigkeitskonzentrat ist, wird die Dialysevorrichtung derart gesteuert, dass die Restmenge an Dialysierflüssigkeitskonzentrat in dem letzten Behandlungszyklus vollständig verbraucht wird oder eine vorgegebene Restmenge an Dialysierflüssigkeitskonzentrat in dem Behältnis verbleibt. Beispielsweise kann eine konstante Dialysierflüssigkeitsrate so eingestellt werden, dass die Restmenge möglichst vollständig verbraucht wird. Es kann aber auch ein entsprechendes Profil für die Dialysierflüssigkeitsrate vorgegeben werden. Damit wird die Dialysedosis für den letzten Patienten erhöht. Die Dialysedosis kann aber für sämtliche Patienten nicht verringert werden.

Eine alternative Ausführungsform sieht nach der Durchführung der einzelnen Behandlungszyklen nicht eine Überprüfung vor, ob die in dem Behältnis noch enthaltende Konzentratmenge für sämtliche noch durchzuführende Behandlungszyklen ausreichend ist, sondern nur für die nächste Behandlung ausreichend ist. Folglich wird ein Alarmsignal erzeugt, wenn die Restmenge an Dialysierflüssigkeitskonzentrat kleiner als die für den nachfolgenden Behandlungszyklus vorgesehene Menge an Dialysierflüssigkeitskonzentrat ist, und ein Steuersignal wird erzeugt, wenn die Restmenge an Dialysierflüssigkeitskonzentrat nicht kleiner als die für den nachfolgenden Behandlungszyklus vorgesehene Menge an Dialysierflüssigkeitskonzentrat ist.

Bei dem Ausführungsbeispiel wird beispielsweise nach der Durchführung des ersten Behandlungszyklus x=1 und vor der Durchführung des zweiten Behandlungszyklus x=2 überprüft, ob das für die noch durchzuführenden Behandlungszyklen zur Verfügung stehende Restvolumen an Dialysierflüssigkeitskonzentrat für den zweiten Behandlungszyklus ausreichend ist. Hierzu wird die tatsächlich verbrauchte Menge $V_{1,ist}$ an Dialysierflüssigkeitskonzentrat ermittelt und die für den zweiten und dritten Behandlungszyklus noch zur Verfügung stehende Restmenge $M_1$ an Dialysierflüssigkeitskonzentrat in dem Behältnis berechnet:

$$M_1 = M_0 - V_{1,ist}$$

Wenn die Restmenge $M_1$ an Dialysierflüssigkeitskonzentrat kleiner als die für den zweiten Behandlungszyklus vorgesehene Menge $V_2$ an Dialysierflüssigkeitskonzentrat ist, wird ein Alarmsignal erzeugt. Ansonsten wird ein Steuersignal zur Einleitung des zweiten Behandlungszyklus erzeugt.

Bei einer weiteren alternativen Ausführungsform ist die Steuer- und Auswerteinheit 24 derart konfiguriert, dass vor der Durchführung der Behandlungszyklen die in dem Behältnis enthaltenden Menge $M_0$ an Dialysierflüssigkeitskonzentrat auf die vorgegebene Anzahl x von Behandlungszyklen aufgeteilt wird, wobei den Behandlungszyklen jeweils eine vorgegebene Menge $V_1'$, $V_2'$, $V_3'$ ... $V_x'$ an Dialysierflüssigkeitskonzentrat zugeteilt wird, die derart bemessen ist, dass nach der Durchführung der Behandlungszyklen in dem Behältnis eine vorgegebene Restmenge $M_R$ oder keine Restmenge an Dialysierflüssigkeitskonzentrat verbleibt.

Die in dem Behältnis zur Verfügung stehende Menge $M_0$ an Dialysierflüssigkeitskonzentrat wird nach der Bestimmung der für die einzelnen Behandlungszyklen vorgesehenen Konzentratmengen $V_1$, $V_2$...$V_x$ so aufgeteilt, dass $M_0$ nach dem letzten Zyklus vollständig oder fast vollständig verbraucht ist. Die Aufteilung der Konzentratmengen auf die Zyklen (x=1-4) kann zu gleichen Teilen $M_0/x$ oder prozentual entsprechend des vorgesehenen Konzentratverbrauchs $V_1$, $V_2$...$V_x$ erfolgen, woraus sich dann die pro Zyklus zur Verfügung stehende Konzentratmenge ergibt:

$$V_1`, V_2`...V_x`$$

mit der Maßgabe

$$V_1` >= V_1; V_2` >= V_2 ; ... ;V_x` >= V_x$$

und

$$V_1`+V_2`+ ... V_x`= M_0 \text{ bzw. } V_1`+V_2`+ ... V_x`= M_0 - M_k$$

Für jeden Behandlungszyklus steht somit eine größere Menge an Dialysierflüssigkeitskonzentrat zur Verfügung, so dass die Dialysedosis für sämtliche Patienten erhöht wird. Wenn aber tatsächlich mehr Dialysierflüssigkeitskonzentrat verbraucht werden sollte als vorgesehen, wird Alarm gegeben.

Bei dem vorliegenden Ausführungsbeispiel werden für die 3 Behandlungszyklen folgende Konzentratmengen bestimmt:

$$V_1=V_{Test}+V_{Beh}+V_{Rein(optional)} \text{ (Behandlungsparamter für Zyklus 1)}$$

$$V_2=V_{Test}+V_{Beh}+V_{Rein(optional)} \text{ (Behandlungsparamter für Zyklus 2)}$$

$$V_3=V_{Test}+V_{Beh}+V_{Rein(optional)} \text{ (Behandlungsparamter für Zyklus 3)}$$

$$V_1`, V_2`, V_3`$$

mit der Maßgabe

$$V_1` >= V_1; V_2` >= V_2 ; V_3` >= V_3$$

und

$$V_1`+V_2`+ V_3`= M_0 \text{ bzw. } V_1`+V_2`+ V_3`= M_0 - M_k$$

Die Dialysevorrichtung wird derart gesteuert, dass die für den ersten Behandlungszyklus vorgegebene Menge $V_1'$ an Dialysierflüssigkeitskonzentrat verbraucht wird. Hierzu kann eine konstante Dialysierflüssigkeitsrate entsprechend angepasst oder das Profil entsprechend verändert werden.

Nach dem ersten Behandlungszyklus mit der für den ersten Behandlungszyklus vorgegebenen Menge $V_1'$ an Dialysierflüssigkeitskonzentrat wird vor Beginn des zweiten Behandlungszyklus mit der Konzentratmenge $V_2'$ die in dem ersten Behandlungszyklus tatsächlich verbrauchte Menge $V_{1,ist}$ an Dialysierflüssigkeitskonzentrat ermittelt, um das für den zweiten und dritten Behandlungszyklus zur Verfügung stehende Restvolumen an Dialysierflüssigkeitskonzentrat aus der Differenz der in dem Behältnis enthaltenden Menge an Dialysierflüssigkeitskonzentrat und der in dem ersten Behandlungszyklus tatsächlich verbrauchten Menge an Dialysierflüssigkeitskonzentrat zu berechnen.

Das jeweils für die noch durchzuführenden Behandlungszyklen zur Verfügung stehende Restvolumen an Dialysierflüssigkeitskonzentrat wird nunmehr wieder auf die noch durchzuführende Anzahl von Behandlungszyklen aufgeteilt, wobei den Behandlungszyklen jeweils wieder eine vorgegebene Menge $V_1''$, $V_2''$, $V_3''$ ... $V_x''$ an Dialysierflüssigkeitskonzentrat zugeteilt wird, die derart bemessen ist, dass nach der Durchführung der Behandlungszyklen in dem Behältnis eine vorgegebene Restmenge $M_k$ oder keine Restmenge an Dialysierflüssigkeitskonzentrat verbleibt. Diese Aufteilung des Dialysierflüssigkeitskonzentrat erfolgt sukzessive bis zu dem letzten Behandlungszyklus. Bei dem Ausführungsbeispiel wird die Konzentratrestmenge nach dem ersten Zyklus auf den zweiten und dritten Zyklus aufgeteilt, d. h. die für den zweiten und dritten Zyklus vorgesehen Konzentratmenge wird um den verbleibenden Überschuss erhöht. Nach dem zweiten Behandlungszyklus steht die Restmenge dann für den dritten Zyklus zur Verfügung.

Die beschriebenen Verfahrensschritte werden sukzessive so lange durchgeführt, bis sämtliche Behandlungen an dem Dialysetag abgeschlossen sind. Der Konzentratbeutel 13B bleibt solange an der Dialysevorrichtung angeschlossen, bis sämtliche Behandlungen abgeschlossen sind. Erst zum Ende des Dialysetags wird der Beutel entfernt, wobei schon jetzt ein neuer Beutel für den nächsten Dialysetag an die Anschlusseinheit angeschlossen werden kann.

Während der Konzentratbeutel 13B an die Dialysevorrichtung angeschlossen ist, erfolgt zwischen den einzelnen Dialysebehandlungen eine Desinfektion der Dialysevorrichtung. Hierzu wird die Desinfektionseinheit 58 in Betrieb gesetzt, wobei die Absperrorgane 56 und 47 in der Dialysierflüssigkeitszuführ- und -abführleitung 8, 9 geschlossen werden. Dadurch erfolgt eine Desinfektion nur in dem ersten Abschnitt IIA des Dialysierflüssigkeitssystems II. In dem zweiten Abschnitt IIB des Dialysierflüssigkeitssystems I wird die Dialysierflüssigkeit für die nachfolgende Behandlung aufbereitet, während die Desinfektionsroutine durchgeführt wird, so dass mit der nachfolgenden Behandlung unmittelbar nach dem Ende der Desinfektionsroutine begonnen werden kann. Eine Desinfektion des gesamten Dialysierflüssigkeitssystems erfolgt erst zum Ende des Dialysetags, so dass die Dialysevorrichtung am nächsten Dialysetag wieder sofort betriebsbereit ist.

**Patentansprüche**

1. Vorrichtung zur Versorgung einer Dialysevorrichtung mit Dialysierflüssigkeit mit
   einer Anschlusseinheit (50) zum Anschließen eines zur einmaligen Verwendung bestimmten Behältnisses (13), das mit einem Dialysierflüssigkeitskonzentrat (K2) für die Herstellung von Dialysierflüssigkeit befüllt ist, wobei die Menge ($M_0$) des in dem Behältnis enthaltenden Dialysierflüssigkeitskonzentrats derart bemessen ist, dass mit dem Dialysierflüssigkeitskonzentrat eine für eine vorgegebene Anzahl (x) von Behandlungszyklen ausreichende Menge an Dialysierflüssigkeit herstellbar ist,
   einer Eingabeeinheit (24A) zur Eingabe von Behandlungsparametern für jeden Behandlungszyklus der vorgegebenen Anzahl (x) von Behandlungszyklen,
   **dadurch gekennzeichnet, dass** eine Rechen- und Auswerteinheit (24) vorgesehen ist, die derart konfiguriert ist, dass
   eine für den Behandlungszyklus vorgesehene Menge ($V_1$, $V_2$, $V_3$, ... $V_x$) an Dialysierflüssigkeitskonzentrat für jeden Behandlungszyklus der vorgegebenen Anzahl (x) von Behandlungszyklen auf der Grundlage der eingegebenen Behandlungsparameter bestimmt wird,
   die Differenz von der in dem Behältnis enthaltenden Menge ($M_0$) an Dialysierflüssigkeitskonzentrat und der für die vorgegebene Anzahl (x) von Behandlungszyklen vorgesehenen Menge ($V_1 + V_2 + V_3 ... + V_x$) an Dialysierflüssigkeitskonzentrat berechnet wird, wobei
   ein Alarmsignal zur Erzeugung eines Alarms erzeugt wird, wenn die in dem Behältnis enthaltende Menge ($M_0$) an Dialysierflüssigkeitskonzentrat kleiner als die für die vorgegebene Anzahl von Behandlungszyklen vorgesehene Menge ($V_1 + V_2 + V_3 ... + V_x$) an Dialysierflüssigkeitskonzentrat ist, und
   ein Steuersignal zur Einleitung des ersten Behandlungszyklus erzeugt wird, wenn die in dem Behältnis enthaltende Menge ($M_0$) an Dialysierflüssigkeitskonzentrat nicht kleiner als die für die vorgegebene Anzahl von Behandlungszyklen vorgesehene Menge ($V_1 + V_2 + V_3 ... + V_x$) an Dialysierflüssigkeitskonzentrat ist.

2. Vorrichtung zur Versorgung einer Dialysevorrichtung mit Dialysierflüssigkeit nach Anspruch 1, **dadurch gekennzeichnet, dass** die Rechen- und Auswerteinheit (24) derart konfiguriert ist, dass
   nach der Durchführung des ersten Behandlungszyklus mit der für den ersten Behandlungszyklus vorgesehenen Menge an Dialysierflüssigkeitskonzentrat jeweils vor Beginn eines nachfolgenden Behandlungszyklus mit der für den jeweiligen nachfolgenden Behandlungszyklus vorgesehenen Menge an Dialysierflüssigkeitskonzentrat
   die in dem vorausgehenden Behandlungszyklus tatsächlich verbrauchte Menge ($V_{ist}$) an Dialysierflüssigkeitskonzentrat ermittelt wird,
   das für die noch durchzuführenden Behandlungszyklen zur Verfügung stehende Restvolumen an Dialysierflüssigkeitskonzentrat aus der Differenz der vor der Durchführung des vorausgehenden Behandlungszyklus in dem Behältnis enthaltenden Menge an Dialysierflüssigkeitskonzentrat und der in dem vorausgehenden Behandlungszyklus tatsächlich verbrauchten Menge an Dialysierflüssigkeitskonzentrat berechnet wird, und
   ein Alarmsignal für einen Alarm erzeugt wird, wenn die Restmenge an Dialysierflüssigkeitskonzentrat kleiner als die für die noch durchzuführenden Behandlungszyklen vorgesehene Menge an Dialysierflüssigkeitskonzentrat ist, und

ein Steuersignal zur Einleitung der nachfolgenden Dialysebehandlung erzeugt wird,
wenn die Restmenge an Dialysierflüssigkeitskonzentrat nicht kleiner als die für die noch durchzuführenden Behandlungszyklen vorgesehene Menge an Dialysierflüssigkeitskonzentrat ist.

3. Vorrichtung zur Versorgung einer Dialysevorrichtung mit Dialysierflüssigkeit nach Anspruch 1, **dadurch gekennzeichnet, dass** die Rechen- und Auswerteinheit (24) derart konfiguriert ist, dass
nach der Durchführung des ersten Behandlungszyklus jeweils vor Beginn eines nachfolgenden Behandlungszyklus die in dem vorausgehenden Behandlungszyklus tatsächlich verbrauchte Menge ($V_{ist}$) an Dialysierflüssigkeitskonzentrat ermittelt wird,
das für die noch durchzuführenden Behandlungszyklen zur Verfügung stehende Restvolumen an Dialysierflüssigkeitskonzentrat aus der Differenz der vor der Durchführung des vorausgehenden Behandlungszyklus in dem Behältnis enthaltenden Menge an Dialysierflüssigkeitskonzentrat und der in dem vorausgehenden Behandlungszyklus tatsächlich verbrauchten Menge an Dialysierflüssigkeitskonzentrat berechnet wird, und
ein Alarmsignal für einen Alarm erzeugt wird, wenn die Restmenge an Dialysierflüssigkeitskonzentrat kleiner als die für den nachfolgenden Behandlungszyklus vorgesehene Menge an Dialysierflüssigkeitskonzentrat ist, und
dass ein Steuersignal zur Einleitung der nachfolgenden Dialysebehandlung erzeugt wird, wenn die Restmenge an Dialysierflüssigkeitskonzentrat nicht kleiner als die für den nachfolgenden Behandlungszyklus vorgesehene Menge an Dialysierflüssigkeitskonzentrat ist.

4. Vorrichtung zur Versorgung einer Dialysevorrichtung mit Dialysierflüssigkeit nach Anspruch 2 oder 3, **dadurch gekennzeichnet, dass** die Rechen- und Auswerteinheit (24) derart konfiguriert ist, dass
für die Dialysevorrichtung ein Steuersignal erzeugt wird, wenn die Restmenge an Dialysierflüssigkeitskonzentrat größer als die für den letzten Behandlungszyklus vorgesehene Menge an Dialysierflüssigkeitskonzentrat ist, mit dem die Dialysevorrichtung derart gesteuert wird, dass die Restmenge an Dialysierflüssigkeitskonzentrat in dem letzten Behandlungszyklus vollständig verbraucht wird oder eine vorgegebene Restmenge an Dialysierflüssigkeitskonzentrat in dem Behältnis verbleibt.

5. Vorrichtung zur Versorgung einer Dialysevorrichtung mit Dialysierflüssigkeit nach Anspruch 1, **dadurch gekennzeichnet, dass** die Rechen- und Auswerteinheit (24) derart konfiguriert ist, dass
vor der Durchführung der Behandlungszyklen die in dem Behältnis enthaltenden Menge ($M_0$) an Dialysierflüssigkeitskonzentrat auf die vorgegebene Anzahl x von Behandlungszyklen aufgeteilt wird, wobei den Behandlungszyklen jeweils eine vorgegebene Menge ($V_1'$, $V_2'$, $V_3'$ ... $V_x'$) an Dialysierflüssigkeitskonzentrat zugeteilt wird, die derart bemessen ist, dass nach der Durchführung der Behandlungszyklen in dem Behältnis eine vorgegebene Restmenge oder keine Restmenge an Dialysierflüssigkeitskonzentrat verbleibt.

6. Vorrichtung zur Versorgung einer Dialysevorrichtung mit Dialysierflüssigkeit nach Anspruch 5, **dadurch gekennzeichnet, dass** die Rechen- und Auswerteinheit (24) derart konfiguriert ist, dass
für die Dialysevorrichtung ein Steuersignal erzeugt wird, mit dem die Dialysevorrichtung derart gesteuert wird, dass die für den ersten Behandlungszyklus vorgegebene Menge ($V_1'$) an Dialysierflüssigkeitskonzentrat verbraucht wird.

7. Vorrichtung zur Versorgung einer Dialysevorrichtung mit Dialysierflüssigkeit nach Anspruch 6, **dadurch gekennzeichnet, dass** die Rechen- und Auswerteinheit (24) derart konfiguriert ist, dass
nach der Durchführung des ersten Behandlungszyklus mit der für den ersten Behandlungszyklus vorgegebenen Menge an Dialysierflüssigkeitskonzentrat jeweils vor Beginn eines nachfolgenden Behandlungszyklus mit der für den jeweiligen nachfolgenden Behandlungszyklus vorgegebenen Menge an Dialysierflüssigkeitskonzentrat
die in dem vorausgehenden Behandlungszyklus tatsächlich verbrauchte Menge ($V_{ist}$) an Dialysierflüssigkeitskonzentrat ermittelt wird,
das für die noch durchzuführenden Behandlungszyklen zur Verfügung stehende Restvolumen an Dialysierflüssigkeitskonzentrat aus der Differenz der vor der Durchführung des vorausgehenden Behandlungszyklus in dem Behältnis enthaltenden Menge an Dialysierflüssigkeitskonzentrat und der in dem vorausgehenden Behandlungszyklus tatsächlich verbrauchten Menge an Dialysierflüssigkeitskonzentrat berechnet wird, und
das jeweils für die noch durchzuführenden Behandlungszyklen zur Verfügung stehende Restvolumen an Dialysierflüssigkeitskonzentrat auf die noch durchzuführende Anzahl von Behandlungszyklen aufgeteilt wird, wobei den Behandlungszyklen jeweils eine vorgegebene Menge $V_1''$, $V_2''$, $V_3''$ ... $V_x''$ an Dialysierflüssigkeitskonzentrat zugeteilt wird, die derart bemessen ist, dass nach der Durchführung der Behandlungszyklen in dem Behältnis eine vorgegebene Restmenge oder keine Restmenge an Dialysierflüssigkeitskonzentrat verbleibt.

8. Vorrichtung zur Versorgung einer Dialysevorrichtung mit Dialysierflüssigkeit nach Anspruch 7, **dadurch gekenn-**

**zeichnet, dass** die Rechen- und Auswerteinheit (24) derart konfiguriert ist, dass
ein Alarmsignal zur Erzeugung eines Alarms erzeugt wird, wenn das jeweils für die noch durchzuführenden Behandlungszyklen zur Verfügung stehende Restvolumen an Dialysierflüssigkeitskonzentrat kleiner als die für die noch durchzuführenden Behandlungszyklen vorgegebene Menge an Dialysierflüssigkeitskonzentrat ist, und
ein Steuersignal zur Einleitung des nachfolgenden Behandlungszyklus erzeugt wird, wenn das jeweils für die noch durchzuführenden Behandlungszyklen zur Verfügung stehende Restvolumen an Dialysierflüssigkeitskonzentrat nicht kleiner als die für die noch durchzuführenden Behandlungszyklen vorgegebene Menge an Dialysierflüssigkeitskonzentrat ist.

9. Vorrichtung zur Versorgung einer Dialysevorrichtung mit Dialysierflüssigkeit nach einem der Ansprüche 1 bis 8, **dadurch gekennzeichnet, dass** der Behandlungszyklus eine der Dialysebehandlung vorausgehende Vorbereitungsphase zur Vorbereitung der Dialysebehandlung und eine Behandlungsphase zur Durchführung der Dialysebehandlung umfasst, wobei die für einen Behandlungszyklus vorgesehene Menge an Dialysierflüssigkeitskonzentrat eine vorgegebene Menge an Dialysierflüssigkeitskonzentrat für die Vorbereitungsphase und eine vorgegebene Menge an Dialysierflüssigkeitskonzentrat für die Behandlungsphase umfasst.

10. Vorrichtung zur Versorgung einer Dialysevorrichtung mit Dialysierflüssigkeit nach einem der Ansprüche 1 bis 9, **dadurch gekennzeichnet, dass** das Behältnis ein Beutel oder eine Kartusche ist, wobei das Volumen des Beutels oder der Kartusche derart bemessen ist, dass der Beutel oder die Kartusche eine für eine vorgegebene Anzahl (x) von Behandlungszyklen ausreichende Menge an Dialysierflüssigkeitsonzentrat aufnimmt.

11. Dialysevorrichtung mit einer Vorrichtung zur Versorgung der Dialysevorrichtung mit Dialysierflüssigkeit nach einem der Ansprüche 1 bis 10.

## Claims

1. Device for supplying a dialysis device with dialysate, comprising
a connecting unit (50) for connecting a container (13) that is intended for single use and is filled with a dialysate concentrate (K2) for producing dialysate, wherein the amount ($M_0$) of dialysate concentrate in the container is set in such a way that it is possible to use the dialysate concentrate to produce an amount of dialysate sufficient for a specified number (x) of treatment cycles,
an input unit (24A) for inputting treatment parameters for each treatment cycle of the specified number (x) of treatment cycles,
**characterised in that** an arithmetic and evaluation unit (24) is provided, the arithmetic and evaluation unit being configured in such a way that
an amount ($V_1$, $V_2$, $V_3$, ... $V_x$) of dialysate concentrate provided for the treatment cycle is determined for each treatment cycle of the specified number (x) of treatment cycles on the basis of the inputted treatment parameters,
the difference between the amount ($M_0$) of dialysate concentrate in the container and the amount ($V_1 + V_2 + V_3 ... + V_x$) of dialysate concentrate provided for the specified number (x) of treatment cycles is calculated, wherein
an alarm signal for generating an alarm is generated if the amount ($M_0$) of dialysate concentrate in the container is less than the amount ($V_1 + V_2 + V_3... + V_x$) of dialysate concentrate provided for the specified number of treatment cycles, and
a control signal for initiating the first treatment cycle is generated if the amount ($M_0$) of dialysate concentrate in the container is not less than the amount ($V_1 + V_2 + V_3... + V_x$) of dialysate concentrate provided for the specified number of treatment cycles.

2. Device for supplying a dialysis device with dialysate according to claim 1, **characterised in that** the arithmetic and evaluation unit (24) is configured in such a way that
after the first treatment cycle has been carried out using the amount of dialysate concentrate provided for the first treatment cycle and before the start of each subsequent treatment cycle using the amount of dialysate concentrate provided for each of the subsequent treatment cycles,
the amount ($V_{actual}$) of dialysate concentrate actually used up in the preceding treatment cycle is determined,
the residual volume of dialysate concentrate available for the treatment cycles still to be carried out is calculated from the difference between the amount of dialysate concentrate in the container before the preceding treatment cycle was carried out and the amount of dialysate concentrate actually used up in the preceding treatment cycle, and
an alarm signal for an alarm is generated if the residual amount of dialysate concentrate is less than the amount of dialysate concentrate provided for the treatment cycles still to be carried out, and

a control signal for initiating the following dialysis treatment is generated if the residual amount of dialysate concentrate is not less than the amount of dialysate concentrate provided for the treatment cycles still to be carried out.

3. Device for supplying a dialysis device with dialysate according to claim 1, **characterised in that** the arithmetic and evaluation unit (24) is configured in such a way that

after the first treatment cycle has been carried out and before the start of each subsequent treatment cycle,

the amount ($V_{actual}$) of dialysate concentrate actually used up in the preceding treatment cycle is determined,

the residual volume of dialysate concentrate available for the treatment cycles still to be carried out is calculated from the difference between the amount of dialysate concentrate in the container before the preceding treatment cycle was carried out and the amount of dialysate concentrate actually used up in the preceding treatment cycle, and

an alarm signal for an alarm is generated if the residual amount of dialysate concentrate is less than the amount of dialysate concentrate provided for the following treatment cycle, and

a control signal for initiating the following dialysis treatment is generated if the residual amount of dialysate concentrate is not less than the amount of dialysate concentrate provided for the following treatment cycle.

4. Device for supplying a dialysis device with dialysate according to either claim 2 or claim 3, **characterised in that** the arithmetic and evaluation unit (24) is configured in such a way that

if the residual amount of dialysate concentrate is greater than the amount of dialysate concentrate provided for the final treatment cycle, a control signal is generated for the dialysis device, by means of which signal the dialysis device is controlled in such a way that the residual amount of dialysate concentrate is completely used up in the final treatment cycle or a specified residual amount of dialysate concentrate remains in the container.

5. Device for supplying a dialysis device with dialysate according to claim 4, **characterised in that** the arithmetic and evaluation unit (24) is configured in such a way that

before the treatment cycles are carried out, the amount ($M_0$) of dialysate concentrate in the container is subdivided among the specified number x of treatment cycles, the treatment cycles each being allocated a specified amount ($V_1'$, $V_2'$, $V_3'$... $V_x'$) of dialysate concentrate that is set in such a way that, after the treatment cycles have been carried out, a specified residual amount or no residual amount of dialysate concentrate remains in the container.

6. Device for supplying a dialysis device with dialysate according to claim 5, **characterised in that** the arithmetic and evaluation unit (24) is configured in such a way that

a control signal is generated for the dialysis device, by means of which signal the dialysis device is controlled in such a way that the amount ($V_1'$) of dialysate concentrate specified for the first treatment cycle is used up.

7. Device for supplying a dialysis device with dialysate according to claim 6, **characterised in that** the arithmetic and evaluation unit (24) is configured in such a way that

after the first treatment cycle has been carried out using the amount of dialysate concentrate specified for the first treatment cycle and before the start of each subsequent treatment cycle using the amount of dialysate concentrate specified for each of the subsequent treatment cycles,

the amount ($V_{actual)}$ of dialysate concentrate actually used up in the preceding treatment cycle is determined,

the residual volume of dialysate concentrate available for the treatment cycles still to be carried out is calculated from the difference between the amount of dialysate concentrate in the container before the preceding treatment cycle was carried out and the amount of dialysate concentrate actually used up in the preceding treatment cycle, and

the residual volume of dialysate concentrate available for each of the treatment cycles still to be carried out is subdivided among the number of treatment cycles still to be carried out, the treatment cycles each being allocated a specified amount $V_1''$, $V_2''$, $V_3''$... $V_x''$ of dialysate concentrate that is set in such a way that, after the treatment cycles have been carried out, a specified residual amount or no residual amount of dialysate concentrate remains in the container.

8. Device for supplying a dialysis device with dialysate according to claim 7, **characterised in that** the arithmetic and evaluation unit (24) is configured in such a way that

an alarm signal for generating an alarm is generated if the residual volume of dialysate concentrate available for each of the treatment cycles still to be carried out is less than the amount of dialysate concentrate specified for the treatment cycles still to be carried out, and

a control signal for initiating the following treatment cycle is generated if the residual volume of dialysate concentrate available for each of the treatment cycles still to be carried out is not less than the amount of dialysate concentrate specified for the treatment cycles still to be carried out.

**9.** Device for supplying a dialysis device with dialysate according to any of claims 1 to 8, **characterised in that** the treatment cycle comprises a preparation phase, preceding the dialysis treatment, for preparing the dialysis treatment and a treatment phase for carrying out the dialysis treatment, the amount of dialysate concentrate provided for one treatment cycle comprising a specified amount of dialysate concentrate for the preparation phase and a specified amount of dialysate concentrate for the treatment phase.

**10.** Device for supplying a dialysis device with dialysate according to any of claims 1 to 9, **characterised in that** the container is a bag or cartridge, the volume of the bag or cartridge being set in such a way that the bag or cartridge holds an amount of dialysate concentrate sufficient for a specified number (x) of treatment cycles.

**11.** Dialysis device comprising a device for supplying the dialysis device with dialysate according to any of claims 1 to 10.

**Revendications**

**1.** Dispositif pour alimenter un dispositif de dialyse avec un liquide de dialyse avec une unité de raccordement (50) pour le raccordement d'un récipient (13) destiné à un usage unique, qui est rempli d'un concentré de liquide de dialyse (K2) pour la production d'un liquide de dialyse, dans lequel la quantité ($M_0$) du concentré de liquide de dialyse contenu dans le récipient est dimensionnée de telle sorte qu'une quantité de liquide de dialyse suffisante pour un nombre (x) prédéfini de cycles de traitement peut être produite avec le concentré de liquide de dialyse, une unité d'entrée (24A) pour l'entrée de paramètres de traitement pour chaque cycle de traitement du nombre (x) prédéfini de cycles de traitement, **caractérisé en ce qu'**une unité de calcul et d'évaluation (24) est prévue, qui est configurée de telle sorte que une quantité ($V_1$, $V_2$, $V_3$, ..., $V_x$) de concentré de liquide de dialyse prévue pour le cycle de traitement est définie pour chaque cycle de traitement du nombre (x) prédéfini de cycles de traitement sur la base des paramètres de traitement entrés, la différence de la quantité ($M_0$) de concentré de liquide de dialyse contenu dans le récipient et de la quantité ($V_1 + V_2 + V_3... + V_x$) de concentré de liquide de dialyse prévue pour le nombre (x) prédéfini de cycles de traitement est calculée, dans lequel un signal d'alarme pour générer une alarme est généré, lorsque la quantité ($M_0$) de concentré de liquide de dialyse contenu dans le récipient est inférieure à la quantité ($V_1 + V_2 + V_3... + V_x$) de concentré de liquide de dialyse prévue pour le nombre prédéfini de cycles de traitement, et un signal de commande pour déclencher le premier cycle de traitement est généré, lorsque la quantité ($M_0$) de concentré de liquide de dialyse contenu dans le récipient n'est pas inférieure à la quantité ($V_1 + V_2 + V_3... + V_x$) de concentré de liquide de dialyse prévue pour le nombre prédéfini de cycles de traitement.

**2.** Dispositif pour alimenter un dispositif de dialyse avec un liquide de dialyse selon la revendication 1, **caractérisé en ce que** l'unité de calcul et d'évaluation (24) est configurée de telle sorte que après la réalisation du premier cycle de traitement avec la quantité de concentré de liquide de dialyse prévue pour le premier cycle de traitement respectivement avant le début d'un cycle de traitement suivant avec la quantité de concentré de liquide de dialyse prévue pour le cycle de traitement suivant respectif la quantité ($V_{ist}$) de concentré de liquide de dialyse effectivement consommée dans le cycle de traitement précédent est déterminée, le volume résiduel de concentré de liquide de dialyse à disposition pour les cycles de traitement encore à réaliser est calculé à partir de la différence de la quantité de concentré de liquide de dialyse contenu dans le récipient avant la réalisation du cycle de traitement précédent et de la quantité de concentré de liquide de dialyse effectivement consommée dans le cycle de traitement précédent, et un signal d'alarme pour une alarme est généré, lorsque la quantité résiduelle de concentré de liquide de dialyse est inférieure à la quantité de concentré de liquide de dialyse prévue pour les cycles de traitement encore à réaliser, et un signal de commande pour déclencher le traitement de dialyse suivant est généré, lorsque la quantité résiduelle de concentré de liquide de dialyse n'est pas inférieure à la quantité de concentré de liquide de dialyse prévue pour les cycles de traitement encore à réaliser.

**3.** Dispositif pour alimenter un dispositif de dialyse avec un liquide de dialyse selon la revendication 1, **caractérisé en ce que** l'unité de calcul et d'évaluation (24) est configurée de telle sorte que après la réalisation du premier cycle de traitement respectivement avant le début d'un cycle de traitement suivant la quantité ($V_{ist}$) de concentré de liquide de dialyse effectivement consommée dans le cycle de traitement précédent est déterminée, le volume résiduel de concentré de liquide de dialyse à disposition pour les cycles de traitement encore à réaliser est calculé à partir de la différence de la quantité de concentré de liquide de dialyse contenu dans le récipient avant la réalisation du cycle de traitement précédent et de la quantité de concentré de liquide de dialyse effectivement consommée dans le cycle de traitement précédent, et

un signal d'alarme pour une alarme est généré, lorsque la quantité résiduelle de concentré de liquide de dialyse est inférieure à la quantité de concentré de liquide de dialyse prévue pour le cycle de traitement suivant, et qu'un signal de commande pour déclencher le traitement de dialyse suivant est généré lorsque la quantité résiduelle de concentré de liquide de dialyse n'est pas inférieure à la quantité de concentré de liquide de dialyse prévue pour le cycle de traitement suivant.

4.  Dispositif pour alimenter un dispositif de dialyse avec un liquide de dialyse selon la revendication 2 ou 3, **caractérisé en ce que** l'unité de calcul et d'évaluation (24) est configurée de telle sorte que

un signal de commande est généré pour le dispositif de dialyse, lorsque la quantité résiduelle de concentré de liquide de dialyse est supérieure à la quantité de concentré de liquide de dialyse prévue pour le dernier cycle de traitement, avec lequel le dispositif de dialyse est commandé de telle sorte que la quantité résiduelle de concentré de liquide de dialyse dans le dernier cycle de traitement est entièrement consommée ou qu'il reste une quantité résiduelle prédéfinie de concentré de liquide de dialyse dans le récipient.

5.  Dispositif pour alimenter un dispositif de dialyse avec un liquide de dialyse selon la revendication 1, **caractérisé en ce que** l'unité de calcul et d'évaluation (24) est configurée de telle sorte que

avant la réalisation des cycles de traitement la quantité ($M_0$) de concentré de liquide de dialyse contenu dans le récipient est répartie sur le nombre (x) prédéfini de cycles de traitement, dans lequel respectivement une quantité ($V_1'$, $V_2'$, $V_3'$, ..., $V_x'$) prédéfinie de concentré de liquide de dialyse est attribuée aux cycles de traitement, qui est dimensionnée de telle sorte qu'après la réalisation des cycles de traitement il reste une quantité résiduelle prédéfinie ou aucune quantité résiduelle de concentré de liquide de dialyse dans le récipient.

6.  Dispositif pour alimenter un dispositif de dialyse avec un liquide de dialyse selon la revendication 5, **caractérisé en ce que** l'unité de calcul et d'évaluation (24) est configurée de telle sorte que

un signal de commande pour le dispositif de dialyse est généré, avec lequel le dispositif de dialyse est commandé de telle sorte que la quantité ($V_1'$) de concentré de liquide de dialyse prédéfinie pour le premier cycle de traitement est consommée.

7.  Dispositif pour alimenter un dispositif de dialyse avec un liquide de dialyse selon la revendication 6, **caractérisé en ce que** l'unité de calcul et d'évaluation (24) est configurée de telle sorte que

après la réalisation du premier cycle de traitement avec la quantité de concentré de liquide de dialyse prédéfinie pour le premier cycle de traitement respectivement avant le début d'un cycle de traitement suivant avec la quantité de concentré de liquide de dialyse prédéfinie pour le cycle de traitement suivant respectif la quantité ($V_{ist}$) de concentré de liquide de dialyse effectivement consommée dans le cycle de traitement précédent est déterminée, le volume résiduel de concentré de liquide de dialyse à disposition pour les cycles de traitement encore à réaliser est calculé à partir de la différence de la quantité de concentré de liquide de dialyse contenu dans le récipient avant la réalisation du cycle de traitement précédent et de la quantité de concentré de liquide de dialyse effectivement consommée dans le cycle de traitement précédent, et

le volume résiduel de concentré de liquide de dialyse respectivement à disposition pour les cycles de traitement encore à réaliser est réparti sur le nombre de cycles de traitement encore à réaliser, dans lequel respectivement une quantité ($V_1''$, $V_2''$, $V_3''$, ..., $V_x''$) prédéfinie de concentré de liquide de dialyse est attribuée aux cycles de traitement, qui est dimensionnée de telle sorte qu'après la réalisation des cycles de traitement il reste une quantité résiduelle prédéfinie ou aucune quantité résiduelle de concentré de liquide de dialyse dans le récipient.

8.  Dispositif pour alimenter un dispositif de dialyse avec un liquide de dialyse selon la revendication 7, **caractérisé en ce que** l'unité de calcul et d'évaluation (24) est configurée de telle sorte que

un signal d'alarme pour générer une alarme est généré, lorsque le volume résiduel de concentré de liquide de dialyse respectivement à disposition pour les cycles de traitement encore à réaliser est inférieur à la quantité prédéfinie de concentré de liquide de dialyse prédéfinie pour les cycles de traitement encore à réaliser, et un signal de commande pour déclencher le cycle de traitement suivant est généré, lorsque le volume résiduel de concentré de liquide de dialyse respectivement à disposition pour les cycles de traitement encore à réaliser n'est pas inférieur à la quantité de concentré de liquide de dialyse prédéfinie pour les cycles de traitement encore à réaliser.

9.  Dispositif pour alimenter un dispositif de dialyse avec un liquide de dialyse selon l'une quelconque des revendications 1 à 8, **caractérisé en ce que** le cycle de traitement comprend une phase de préparation précédant le traitement de dialyse pour préparer le traitement de dialyse et une phase de traitement pour réaliser le traitement de dialyse, dans lequel la quantité de concentré de liquide de dialyse prévue pour un cycle de traitement comprend une quantité prédéfinie de concentré de liquide de dialyse pour la phase de préparation et une quantité prédéfinie de concentré de liquide de dialyse pour la phase de traitement.

10. Dispositif pour alimenter un dispositif de dialyse avec un liquide de dialyse selon l'une quelconque des revendications 1 à 9, **caractérisé en ce que** le récipient est un sachet ou une cartouche, dans lequel le volume du sachet ou de la cartouche est dimensionné de telle sorte que le sachet ou la cartouche reçoit une quantité de concentré de liquide de dialyse suffisante pour un nombre (x) prédéfini de cycles de traitement.

11. Dispositif de dialyse avec un dispositif pour alimenter le dispositif de dialyse en liquide de dialyse selon l'une quelconque des revendications 1 à 10.

Fig. 1

# Fig. 2A

# Fig. 2B

**Fig. 2C**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10302691 B3